# EUROPEAN PATENT APPLICATION

(11) **EP 2 873 974 A1**
(43) Date of publication of application: **20.05.2015**
(21) Application number: 13781504.9
(22) Date of filing: 18.03.2013
(51) Int. Cl.: G01N 33/50, G01N 33/53, G01N 33/72

(54) **BIOCHEMICAL ASSAY CARTRIDGE**

(30) Priority: 24.04.2012 KR 20120042774
(71) Applicant: i-Sens, Inc., Nowon-gu Seoul 139-845 (KR)
(72) Inventor: CHA, Guen Sig, Seoul 120-100 (KR); NAM, Hakhyun, Seoul 142-742 (KR); SHEN, Dongxuan, Incheon 406-735 (KR); CHO, Joo Young, Incheon 406-715 (KR); PARK, Kap Soo, Gwangmyeong-si Gyeonggi-do 423-806 (KR); KIM, Jihoon, Gwangmyeong-si Gyeonggi-do 423-060 (KR)
(74) Representative: Office Freylinger
(86) International application number: PCT/KR2013/002182
(87) International publication number: WO 2013/162175

(57) **Abstract**

The present invention relates to a biochemical assay cartridge. More particularly, the present invention provides a biochemical assay cartridge including an insertion-type solution cartridge and a reaction cartridge receiving the insertion-type solution cartridge, in which the solution cartridge is inserted into the reaction cartridge to catch a protruding protection film inducement unit by a latch in the reaction cartridge and thus break the protection film inducement unit and a protection film for sealing a reaction solution storage unit attached to the solution cartridge is automatically detached to discharge the reaction solution from the solution cartridge to the reaction cartridge. The biochemical assay cartridge according to the present invention is automatically injected into the reaction cartridge while a sample to be subjected to assay is inserted, through the insertion-type solution cartridge, into the reaction cartridge, and thus user convenience and operability of the assay cartridge are improved.

## Description

### TECHNICAL FIELD

The present disclosure relates to a biochemical reaction cartridge to optically and simply measure an amount and a concentration of a particular sample through a liquid phase reaction between one or more assay reagents and a small amount of bio-sample, and an assay method thereof, and particularly, to an assay system which may be used in a point-of-care testing (POCT) device.

### BACKGROUND ART

Recently, in a medical diagnosis field, in order to detect or quantify a particular sample included in a bio-sample such as blood, serum, urine, and cell sap, various assay technologies such as enzyme assay, immunoassay, chemical colorimetric assay, electrochemical assay, fluorescence labeling and measurement, and chemiluminescent labeling and measurement have been used. The assay technologies are applied and used in a large apparatus such as an automatic assay device used in a clinical trial center of a hospital, or a point-of-care testing (POCT) device using a platform such as a test strip and a cartridge.

The large automatic apparatus has advantages in that a large quantities of samples can be treated at a high speed and reliability of a measurement value is high, however, there is disadvantages in that the mechanical structure is complicated and the apparatus is used only in a special examination room or a centralized laboratory, and thus an appropriate installation place thereof is limited. Further, since there are many cases where a pre-treatment process of the bio-sample is required and various types of reagents and sensors need to be periodically replaced to be used, it is very cumbersome to perform maintenance and management.

Meanwhile, in the case of the point-of-care testing device, reliability of the measurement value is low as compared to the large apparatus but a measurement is not limited geographically and the measurement can be rapidly performed, and thus the point-of-care testing device is extensively used in the medical diagnosis field. Particularly, unlike the large apparatus in which various types of reagents and sensors are respectively equipped and installed, a cartridge-type point-of-care testing device is constituted by a bio-sample supply unit, a reaction reagent, and a detection unit in one cartridge, and thus convenience for a user is high during measurement. Further, since a possibility of contamination due to exposure of the bio-sample after measurement is low, there is an advantage in terms of safety for medical personnel.

Meanwhile, in diagnosis of diabetes, a demand for point-of-care testing of glycated hemoglobin is on the rise together with blood-sugar measurement. Glycated hemoglobin (HbA1c) is a term meaning a hemoglobin to which a glucose molecule reacts and forms an adduct, and measurement of glycated hemoglobin included in blood may be used as an index of an average blood-sugar value of a patient for the past three to four months regardless of meals and a physical state of the patient and evaluating efficiency of a blood-sugar management method performed by the patient, and thus currently, measurement of glycated hemoglobin receives attention. In practice, until now, many cartridge-type point-of-care testing devices for measuring glycated hemoglobin included in blood are reported and released.

U.S. Patent No. US6,300,142 B1 discloses a cartridge of measuring an assay material by reacting a sample with a first reactant through a first inlet and sequentially reacting the sample with a second reactant through a second inlet in order to measure glycated hemoglobin present in blood. However, in this case, measurements should sequentially occur at temporal intervals and a measurer should intervene in a measurement step so that the samples are sequentially injected to be reacted. Further, there is a problem in that since a reagent solution may be leaked during a measurement process, reliability and marketability of a measurement result are reduced, and there are disadvantages in that since a process of filtering beads combined with glycated hemoglobin once is required, measurement is complicated and required time is long. Moreover, there is a problem in that since the aforementioned works require a user to directly intervene in various steps, the user may feel onerousness and thus a measurement time is naturally delayed.

Further, U.S. Patent No. US7,632,462 B2 discloses an assay cartridge including at least two well spaces and a pipette which may be positioned in at least two well spaces. In this case, the pipette has a stylobate unit and an end unit, and the end unit has a structure which is closed by a membrane that a liquid can permeate. Further, disclosed is an assay device including a holder disposed to receive the cartridge, a driving unit operated to position the pipette in the selected well space in the cartridge, a gas pressure application device combined with the pipette to fluidize the liquid in the pipette through the membrane, a radiation detector detecting radiation from the well space or the pipette of the cartridge to be operated, and an electromagnetic radiation source. The assay device is a cartridge-type point-of-care testing device for glycated hemoglobin having a high quantitative property, but has disadvantages in that structures of the cartridge and the assay device are complicated as described above in order to embody a driving system and thus a cost of the assay device is increased.

Accordingly, the present inventors developed a biochemical assay cartridge including an insertion-type solution cartridge for supplying a bio-sample and a solution reagent and a reaction cartridge having one or more reaction units fixing one or more assay reagents and reacting the assay reagents with the solution reagent and a measurement window performing optical measurement, thereby accomplishing the present invention. In this case, the biochemical assay cartridge according to the present invention includes a unit automatically supplying the bio-sample and the solution reagent while the insertion-type solution cartridge is inserted into the reaction cartridge. Further, a simple driving system continuously inflowing the bio-sample and the solution reagent supplied from the insertion-type solution cartridge along a provided flow path into each reaction unit only by adjusting an angle of the cartridge using gravity was embodied. Accordingly, a biochemical assay cartridge minimizing an intervention process in assay by a measurer has been developed, and a relatively simple and convenient cartridge-type point-of-care testing device was accomplished.

### DISCLOSURE OF THE INVENTION

### TECHNICAL PROBLEM

One object of the present invention is to provide a biochemical assay cartridge.

### TECHNICAL SOLUTION

In order to achieve the object, the present invention provides a biochemical assay cartridge including an insertion-type solution cartridge supplying a sample and a reaction cartridge receiving the insertion-type solution cartridge, in which the insertion-type solution cartridge includes a capillary-type sample storage unit collecting and storing a liquid phase bio-sample and having a sample inlet through which the stored bio-sample is supplied to the reaction cartridge; a reaction solution storage unit storing a reaction solution reacted with the liquid phase bio-sample; a reaction solution protection film attached to prevent the reaction solution from being leaked from the reaction solution storage unit; and a protection film detachment inducement unit attached to an end of the reaction solution protection film and moving toward the reaction solution protection film when the insertion-type solution cartridge is inserted into the reaction cartridge to remove the protection film, and the reaction cartridge includes a receiving unit into which the insertion-type solution cartridge is inserted to be received; a latch coming into contact with the protection film detachment inducement unit when the insertion-type solution cartridge in the receiving unit is inserted into the reaction cartridge to move the protection film detachment inducement unit toward the protection film and thus induce to remove the protection film by the protection film detachment inducement unit; a mixing unit receiving the reaction solution discharged when the protection film is removed and mixing the reaction solution and the bio-sample discharged when the reaction solution comes into contact with a sample inlet of the insertion-type solution cartridge to form a mixed solution; a reagent fixing unit fixing the reagent to induce a reaction of the reagent and the mixed solution of the mixing unit; a measurement unit optically measuring a result of the reaction; and a fine flow path through which the mixing unit, the reagent fixing unit, and the measurement unit are connected.

### ADVANTAGEOUS EFFECTS

A biochemical assay cartridge according to the present invention is advantageous in that a sample to be subjected to assay is automatically injected into a reaction cartridge at the same time when the sample is inserted through an insertion-type solution cartridge into the reaction cartridge, and thus it is possible to improve convenience of a user and operability of the assay cartridge.

### BRIEF DESCRIPTION OF THE DRAWINGS

FIG. 1 is an equiangular perspective view showing a biochemical assay cartridge according to the present invention;
FIG. 2 is a front perspective view showing a lower surface of a reaction cartridge according to the present invention;
FIG. 3 is a front perspective view showing an upper surface of the reaction cartridge according to the present invention;
FIG. 4 is a front perspective view showing a solution cartridge according to the present invention;
FIG. 5 is a view showing a side portion of the solution cartridge according to the present invention;
FIG. 6 is a view showing the solution cartridge according to the present invention in which a reaction solution protection film is attacthed;
FIG. 7 is a view showing a principle that the reaction solution protection film is automatically removed as an insertion-type solution cartridge according to the present invention is inserted; and
FIG. 8 is a schematic view showing an assay process of the biochemical assay cartridge according to the present invention.

### MODE FOR CARRYING OUT THE INVENTION

Hereinafter, the most preferred embodiment of the present invention will be described with reference to the accompanying drawings in order to describe the present invention in detail so that the technical spirit of the present invention can be easily embodied by those skilled in the art to which the present invention belongs. First, it is to be noted that in assigning reference numerals to elements in the drawings, the same reference numerals designate the same elements throughout the drawings although the elements are shown in different drawings. In addition, in the description of the present disclosure, the detailed descriptions of known related constitutions or functions thereof may be omitted if they make the gist of the present invention unclear.

The present invention provides a biochemical assay cartridge including an insertion-type solution cartridge supplying a sample and a reaction cartridge receiving the insertion-type solution cartridge, in which the insertion-type solution cartridge includes a capillary-type sample storage unit collecting and storing a liquid phase bio-sample and having a sample inlet through which the stored bio-sample is supplied to the reaction cartridge; a reaction solution storage unit storing a reaction solution reacted with the liquid phase bio-sample; a reaction solution protection film attached to prevent the reaction solution from being leaked from the reaction solution storage unit; and a protection film detachment inducement unit attached to an end of the reaction solution protection film and moving toward the reaction solution protection film when the insertion-type solution cartridge is inserted into the reaction cartridge to remove the protection film, and the reaction cartridge includes a receiving unit into which the insertion-type solution cartridge is inserted to be received; a latch coming into contact with the protection film detachment inducement unit when the insertion-type solution cartridge in the receiving unit is inserted into the reaction cartridge to move the protection film detachment inducement unit toward the protection film and thus induce to remove the protection film by the protection film detachment inducement unit; a mixing unit receiving the reaction solution discharged when the protection film is removed and mixing the reaction solution and the bio-sample discharged when the reaction solution comes into contact with a sample inlet of the insertion-type solution cartridge to form a mixed solution; a reagent fixing unit fixing the reagent to induce a reaction of the reagent and the mixed solution of the mixing unit; a measurement unit optically measuring a result of the reaction; and a fine flow path through which the mixing unit, the reagent fixing unit, and the measurement unit are connected.

The biochemical assay cartridge according to the present invention will be described in detail through the drawings.

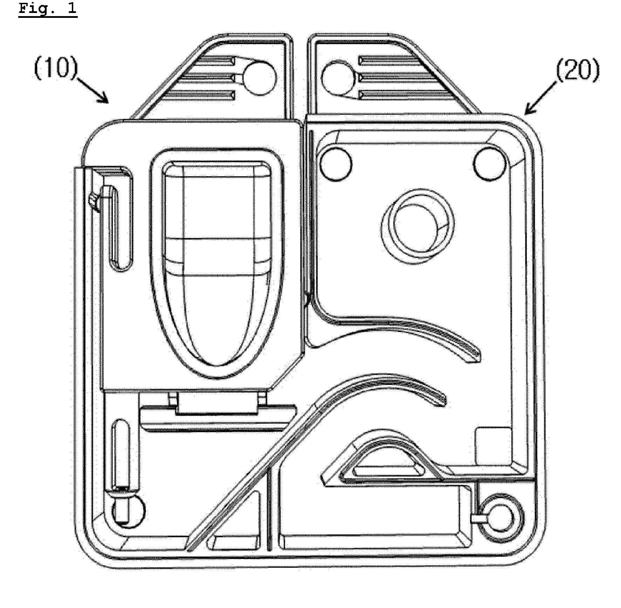

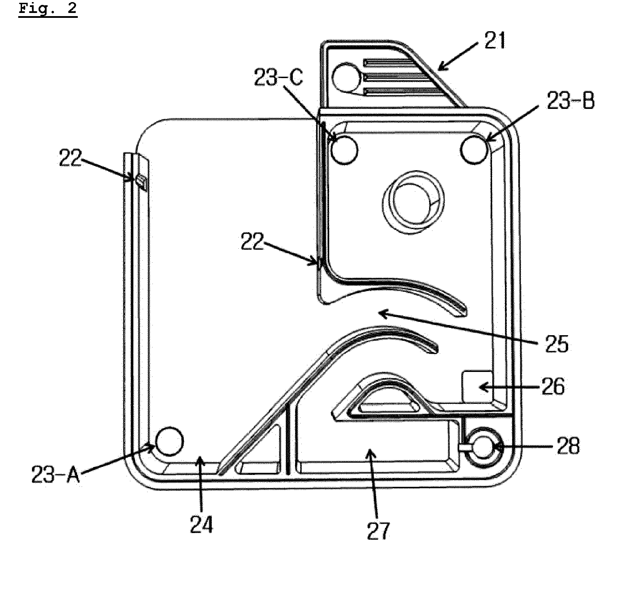

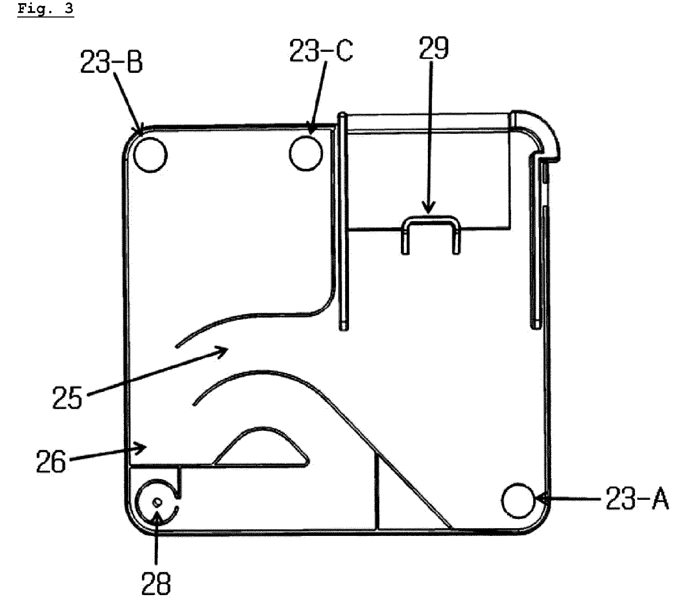

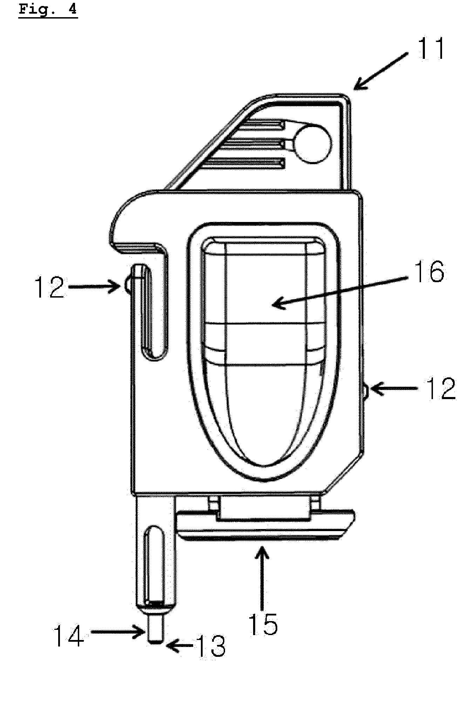

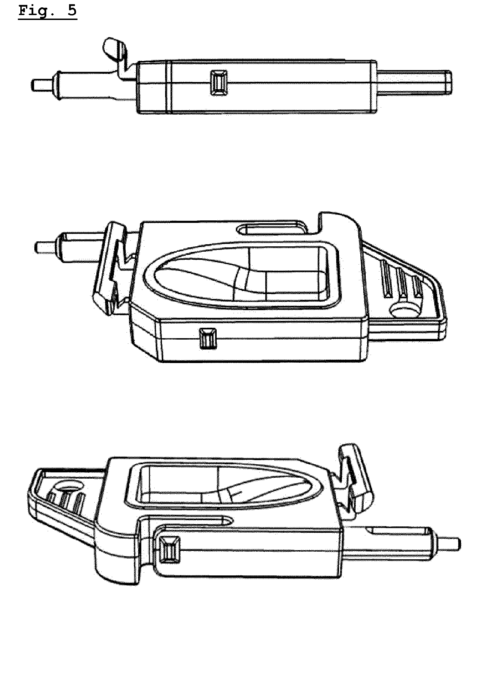

Referring to FIGS. 1 to 5, the biochemical assay cartridge according to the present invention includes an insertion-type solution cartridge 10 for supplying the sample and a reaction cartridge 20 into which the insertion-type solution cartridge is inserted and received. In this case, the insertion-type solution cartridge 10 includes a capillary-type sample storage unit 14 collecting the bio-sample to be subjected to assay in a desired amount and storing the bio-sample, and the bio-sample stored in the sample storage unit is injected through a sample inlet 13 provided in the sample storage unit 14 into the reaction cartridge 20. In this case, the amount of bio-sample may be adjusted by adjusting an internal volume according to an internal diameter and a height of a capillary of the sample storage unit 14, and a structure of the sample storage unit is not limited to a capillary structure as long as the structure is easy to discharge the bio-sample.

Further, the insertion-type solution cartridge 10 includes a reaction solution storage unit 16 storing the reaction solution which may react with the bio-sample, and in order to prevent the reaction solution from being discharged until the insertion-type solution cartridge 10 is inserted into the reaction cartridge 20, a reaction solution protection film 17 is attached to the reaction solution storage unit 16 to seal the reaction solution storage unit.

Moreover, a protection film detachment inducement unit 15 provided in the insertion-type solution cartridge 10 is attached to an end of the reaction solution protection film 17. This is configured to induce automatic detachment of the protection film when the insertion-type solution cartridge 10 is inserted into the reaction cartridge 20. Accordingly, the protection film 17 of the protection film detachment inducement unit 15 is detached while the insertion-type solution cartridge 10 is inserted into the reaction cartridge 20, and thus a process of separately injecting the reaction solution by a user may be omitted.

Meanwhile, the reaction cartridge 20 includes a mixing unit 24 mixing the reaction solution discharged from the solution cartridge 10 and the bio-sample. Further, the reaction cartridge 20 includes one or more reagent fixing units 23 into which a chemical reagent reacted with the reaction solution and the bio-sample mixed in the mixing unit 24 to induce an enzyme reaction and an antigen/antibody reaction, and the bio-sample may be subjected to assay through optical assay of UV/VIS in a measurement region 26 measuring a result of the reaction performed in the reagent fixing units 23.

Moreover, the reaction cartridge 20 may further include a waste liquid treatment unit 27 for collecting a waste liquid measured in the measurement region 26. The waste liquid as a kind of medical wastes may be collected and separately treated through the waste liquid treatment unit 27, and collection of the waste liquid through the waste liquid treatment unit may be performed by adding cotton wool, an absorption filter, and an absorption raw material made of a polymer having strong absorptivity to the waste liquid treatment unit 27 to absorb the waste liquid.

Further, the reaction cartridge 20 may further include an air outlet 28. The air outlet 28 is a structure for smooth movement of the waste liquid and absorption of the absorption raw material, and the waste liquid may be more smoothly moved to the waste liquid treatment unit 27 therethrough and absorption for collecting the waste liquid may be more smoothly performed therethrough.

In this case, the reaction solution and the bio-sample mixed in the mixing unit 24 may be moved through the flow path 25 among the mixing unit 24, one or more reagent fixing units 23, the measurement region 26, and the waste liquid treatment unit 27, and a structure of the flow path 25 is not limited as long as the structure is designed to move the reaction solution and the bio-sample due to gravity when the reaction cartridge 20 is inclined.

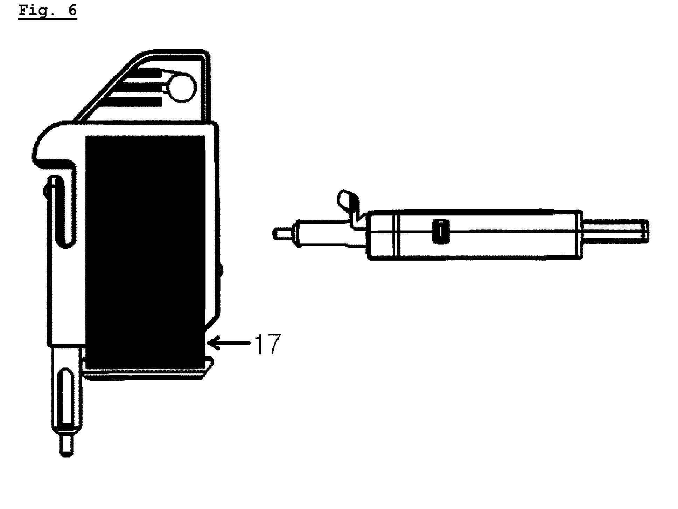

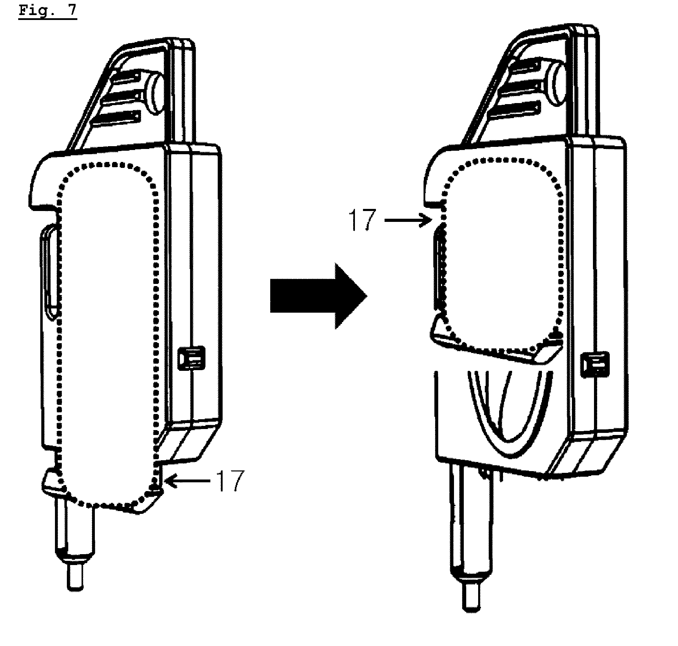

Referring to FIGS. 6 and 7, the reaction solution storage unit 16 of the solution cartridge 10 is sealed by the attached reaction solution protection film 17, and the protection film detachment inducement unit 15 having a protrusion structure provided at a lower end of the reaction solution storage unit 16 is attached to an end of the protection film attached to the reaction solution storage unit 16. Accordingly, when the solution cartridge 10 is inserted into the reaction cartridge 20, the protection film detachment inducement unit 15 comes into contact with a latch 29 provided in the reaction cartridge 20 and is bent in an opposite direction of an insertion direction of the solution cartridge 10 due to the latch 29. Further, if additional force is applied as insertion of the solution cartridge 10 progresses, the protection film detachment inducement unit 15 is broken to be separated from the solution cartridge 10. That is, the protection film detachment inducement unit 15 which is hindered from being inserted due to the latch 29 is bent in the opposite direction of the insertion direction, and the protection film detachment inducement unit is broken due to force applied for additional insertion to be moved in the opposite direction of the insertion direction together with the attached film. As described above, through separation of the solution cartridge 10 and the protection film detachment inducement unit 15, the protection film attached to the film detachment attachment inducement unit 15 and the reaction solution storage unit 16 is automatically detached, and the reaction solution stored in the reaction solution storage unit 16 is discharged due to gravity to be naturally moved to the mixing unit 24 of the reaction cartridge 20.

This series of processes are performed while the insertion-type solution cartridge is inserted into the reaction cartridge, and ultimately, the reaction solution is moved to the mixing unit 24 while the insertion-type solution cartridge is inserted into the reaction cartridge.

As described above, the reaction solution automatically discharged from the reaction solution storage unit 16 by inserting the solution cartridge 10 into the reaction cartridge 20 comes into contact with the sample storage unit 14 of the solution cartridge 10, and the bio-sample in the capillary is discharged due to contact through the sample inlet to the mixing unit 24 of the reaction cartridge 20 to be mixed with the reaction solution. That is, for the biochemical assay cartridge according to the present invention, it can be seen that even though a measurer does not intervene in the injection process of the sample and the injection process of the reaction solution, the bio-sample and the reaction solution are automatically injected and mixed, and thus convenience of a user and operability are excellent.

Meanwhile, the solution cartridge 10 and the reaction cartridge 20 may further include a solution cartridge handle 11 and a reaction cartridge handle 21, respectively. The handle is configured to easily transport and use the solution cartridge 10 and the reaction cartridge 20, and a structure thereof is not particularly limited.

Further, when the solution cartridge 10 is inserted into the reaction cartridge 20, in order to fix the solution cartridge 10, a clamp 12 may be provided on a side of the solution cartridge and a fixing groove 22 may be provided on the receiving unit of the reaction cartridge. The clamp 12 may engage with the fixing groove 22 to fix the inserted solution cartridge 10 to the reaction cartridge 10, and even though the biochemical assay cartridge of the present invention rotates, movement or separation of the solution cartridge according to performing of assay may be prevented.

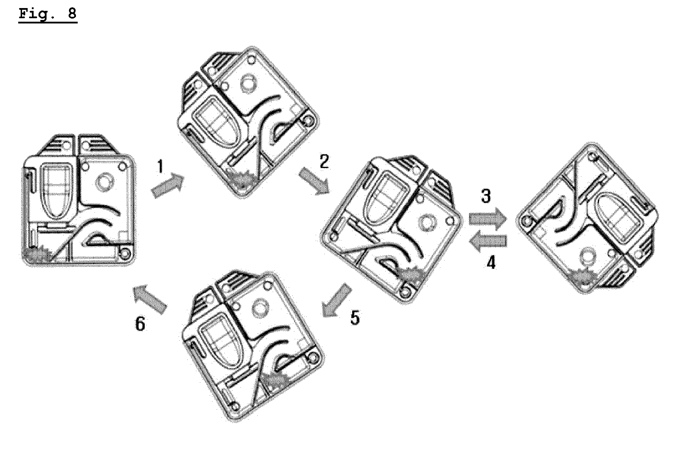

Referring to FIG. 8, assay through the biochemical assay cartridge according to the present invention is as follows.

The solution cartridge 10 is inserted into the reaction cartridge 20 to automatically discharge the reaction solution to the mixing unit 24, the sample storage unit 14 is received in the mixing unit 24, and the discharged reaction solution comes into contact with the sample inlet 13 of the sample storage unit 14 to be mixed with the bio-sample in the sample storage unit. The mixed reaction solution and bio-sample are reacted with a reagent fixed to the reagent fixing unit 23 to be capable of being reacted with the reaction solution and the bio-sample. The reaction may be, for example, an enzyme reaction between a substrate in the mixed solution including the reaction solution and the bio-sample mixed with each other and an enzyme in the reagent, or an antigen-antibody reaction between an antigen in the mixed solution including the reaction solution and the bio-sample mixed with each other and an antibody in the reagent. However, the reaction is not limited thereto, and an appropriate reaction required in optical assay may be induced to perform assay.

A plurality of one or more reagent fixing units 23 may be provided according to an assay target and an assay method, and the result of the reaction performed in the reagent fixing units 23 may be subjected to optical assay in the measurement region 26.

Meanwhile, as sequentially shown in FIG. 8, the reaction solution and the bio-sample mixed in the mixing unit 24 are moved due to gravity along the flow path to the measurement region and the reagent fixing unit according to rotation of the biochemical assay cartridge of the present invention, and thus the reaction and measurement of the reaction solution and the bio-sample are performed. In this case, the structure of the flow path and the assay sequence shown in FIG. 8 is illustrated as an example for performing assay of the bio-sample, and the biochemical assay cartridge according to the present invention is not limited thereto.

The biochemical assay cartridge according to the present invention may be used for the purpose of assay of various bio-samples, preferably the purpose of blood assay, and more preferably the purpose of glycated hemoglobin quantitative assay.

For example, glycated hemoglobin quantitative assay through an enzymatic method using the biochemical assay cartridge according to the present invention will be specifically described.

The enzymatic method for glycated hemoglobin quantitative assay is mostly constituted by four chemical reactions.

A first reaction is a hemolytic reaction of the blood sample, which means a reaction where an erythrocyte of blood is destroyed by using the reaction solution to dissociate hemoglobin. In this case, the reaction solution used for the purpose of the hemolytic reaction may be manufactured by various methods such as adjustment of a pH and use of a surfactant.

A second reaction is a reaction where glycated hemoglobin molecules are cleaved by using a proteolytic enzyme, and various enzymes such as protease A, protease N, dispase, pronase, neutral protease, Glu-C, papain, trypsin, and pepsin may be used.

A third reaction is a reaction where glycated peptide or glycated amino acid molecules generated using cutting by the proteolytic enzyme are oxidated by using an oxidase called FPOX (fructosyl peptide oxidase), and hydrogen peroxide may be generated therethrough.

A fourth reaction is a color reaction, which means a chemical reaction where hydrogen peroxide (H₂O₂) generated through the third reaction is oxidated by using peroxidase (POD) and a color developing reagent and the substrate are reduced due to electrons emitted by oxidation to perform discoloration.

In order to perform glycated hemoglobin quantitative assay through the enzymatic method, the four reactions are performed in the biochemical assay cartridge according to the present invention, and glycated hemoglobin quantitative assay will be described in detail as an example through a structure and an operation method of the cartridge shown in FIGS. 1 to 8.

In order to perform assay, blood is collected and stored in the sample storage unit 14 of the insertion-type solution cartridge 10, and a surfactant which can perform hemolysis of blood is stored as the reaction solution in the reaction solution storage unit 16. The insertion-type solution cartridge 10 is inserted into the reaction cartridge 20 to automatically bring the protruding protection film detachment inducement unit 15 into contact with the latch in the reaction cartridge, the protection film detachment inducement unit is broken according to continuous application of force to be removed from the solution cartridge, and the protection film attached to the reaction solution storage unit is detached together as additional insertion progresses to discharge the reaction solution and move the reaction solution to the mixing unit. The discharged reaction solution and the blood sample are mixed to perform the hemolytic reaction. After the hemolytic reaction is performed, in a first chemical reagent fixing unit 23-A to which one type of reagent of the proteolytic enzyme, FPOX, and POD is fixed, the fixed reagent is dissolved by the blood sample, and the enzyme reaction may be induced therethrough.

The hemolytic reaction is performed in the mixing unit 24, and the reaction solution obtained by finishing the enzyme reaction by the first chemical reagent fixing unit 23-A is moved due to gravity by rotation of the cartridge like FIG. 8 showing an operation principle of the cartridge through the flow path 25 in the reaction cartridge to the measurement region 26. The concentration of total hemolytic hemoglobin of the reaction solution moved to the measurement region is measured by using absorbance exhibited at 535 nm through a UV-Vis spectrophotometer.

The reaction solution obtained by finishing the measurement of total hemoglobin is moved to a second chemical reagent fixing unit 23-B through rotation of the cartridge. The second chemical reagent fixing unit 23-B has a face-to-face structure where a reagent including residual two enzymes other than the enzyme fixed to the first chemical reagent fixing unit 23-A mixed with each other and the color developing reagent are fixed and the reagent of the two enzymes and the color developing reagent face each other.

The reaction solution moved to the second chemical reagent fixing unit 23-B is subjected to both the enzyme reaction and the color reaction, and the reaction solution obtained by finishing the enzyme reaction and the color developing reaction is moved back to the measurement region 26 through rotation of the cartridge. Subsequently, the concentration of glycated hemoglobin colored by the color reaction is measured by using absorbance exhibited around 660 nm through the UV-Vis spectrophotometer.

After assay is finished, the waste liquid is moved to the waste liquid treatment unit 27 by rotation of the cartridge, and the waste liquid is absorbed through the absorption raw material in the waste liquid treatment unit 27 to be collected.

As described above, spectrophotometry needs to be performed twice in order to perform glycated hemoglobin quantitative assay because glycated hemoglobin means a ratio of glycated hemoglobin to the concentration of total hemoglobin. That is, there is onerousness in that an assay process having many steps such as a process of pre-treating a blood sample by a measurer and a process of attaching a marker material needs to be performed. However, when assay of glycated hemoglobin is performed by using the biochemical assay cartridge according to the present invention, as shown in FIG. 8, the enzyme reaction can be performed and absolute concentrations of total hemoglobin and glycated hemoglobin can be measured in one cartridge by moving the reaction solution through rotation of the cartridge. Particularly, the reaction solution (hemolytic reagent during glycated hemoglobin assay) can be automatically discharged to the mixing unit in the reaction cartridge while the insertion-type solution cartridge 10 is inserted into the reaction cartridge 20, and thus direct intervention of a person performing assay in an assay process can be minimized and problems of an assay time delay or a reduction in assay precision can be prevented.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A biochemical assay cartridge including an insertion-type solution cartridge for supplying a sample and a reaction cartridge receiving the insertion-type solution cartridge, wherein
the insertion-type solution cartridge includes:
a capillary-type sample storage unit for collecting and storing a liquid phase bio-sample which has a sample inlet for supplying the stored bio-sample to the reaction cartridge;
a reaction solution storage unit for storing a reaction solution which can react with the liquid phase bio-sample;
a reaction solution protection film attached to prevent the reaction solution from being leaked from the reaction solution storage unit; and
a protection film detachment inducement unit attached to an end of the reaction solution protection film, wherein the protection film detachment inducement unit moves toward the reaction solution protection film as the insertion-type solution cartridge is inserted into the reaction cartridge to remove the protection film, and
the reaction cartridge includes:
a receiving unit into which the insertion-type solution cartridge is inserted to be received;
a latch coming into contact with the protection film detachment inducement unit when the insertion-type solution cartridge in the receiving unit is inserted into the reaction cartridge to move the protection film detachment inducement unit toward the protection film and thus induce to remove the protection film by the protection film detachment inducement unit;
a mixing unit receiving the discharged reaction solution when the protection film is removed and mixing the reaction solution and the dischared bio-sample by contacing the reaction solution with a sample inlet of the insertion-type solution cartridge to form a mixed solution;
a reagent fixing unit for fixing a reagent to induce a reaction of the reagent and the mixed solution of the mixing unit;
a measurement unit for optically measuring a result of the reaction; and
a fine flow path through which the mixing unit, the reagent fixing unit, and the measurement unit are connected.

2. The biochemical assay cartridge as set forth in claim 1, wherein the reaction solution is moved to the mixing unit while the insertion-type solution cartridge is inserted into the reaction cartridge.

3. The biochemical assay cartridge as set forth in claim 1, wherein mixing unit receives the sample storage unit as the insertion-type solution cartridge is inserted into the reaction cartridge.

4. The biochemical assay cartridge as set forth in claim 1, wherein the reaction cartridge includes a plurality of reagent fixing units.

5. The biochemical assay cartridge as set forth in claim 1, wherein the reaction is an enzyme reaction between a substrate in the mixed solution and an enzyme in the reagent, or an antigen-antibody reaction between an antigen in the mixed solution and an antibody in the reagent.

6. The biochemical assay cartridge as set forth in claim 1, wherein the mixed solution of the bio-sample and a chemical reagent is moved along the flow path to a measurement region or the reagent fixing unit by gravity and centrifugal force caused by rotation of the entire biochemical assay cartridge.

7. The biochemical assay cartridge as set forth in claim 1, wherein the reaction cartridge of the biochemical assay cartridge further includes a waste liquid treatment unit for treating the mixed solution of the bio-sample, the reaction solution, and a chemical reagent after measurment.

8. The biochemical assay cartridge as set forth in claim 7, wherein the reaction cartridge of the biochemical assay cartridge further includes an air outlet for smooth movement and collection of a waste liquid.

9. The biochemical assay cartridge as set forth in claim 1, wherein the biochemical assay cartridge is a biochemical assay cartridge for blood assay.

10. The biochemical assay cartridge as set forth in claim 1, wherein the biochemical assay cartridge is a biochemical assay cartridge for glycated hemoglobin quantitative assay.
